(19) Europäisches Patentamt
European Patent Office
Office européen des brevets

(11) Publication number: **0 116 373**
**B1**

# (12) EUROPEAN PATENT SPECIFICATION

(45) Date of publication of patent specification: **15.04.87**

(51) Int. Cl.⁴: **C 07 D 498/04,**
C 07 D 498/14, A 61 K 31/535
// C07D313/08 ,(C07D498/04,
313:00, 265:00),(C07D498/14,
317:00, 313:00, 265:00)

(21) Application number: **84101520.9**

(22) Date of filing: **14.02.84**

## (54) 2H-(1) benzoxepino(5,4-b)-1,4-oxazine derivatives.

(30) Priority: **15.02.83 US 466407**
**21.12.83 US 562759**

(43) Date of publication of application:
**22.08.84 Bulletin 84/34**

(45) Publication of the grant of the patent:
**15.04.87 Bulletin 87/16**

(84) Designated Contracting States:
**AT BE CH DE FR GB IT LI LU NL SE**

(56) References cited:
**GB-A-1 211 258**

(73) Proprietor: **MERRELL DOW PHARMACEUTICALS INC.**
**2110 E. Galbraith Road**
**Cincinnati Ohio 45215 (US)**

(72) Inventor: **Freedman, Jules**
**10553 Adventure Lane**
**Cincinnati, OH 45242 (US)**
Inventor: **Dage, Richard C.**
**7825 Shadowhill Way**
**Cincinnati, OH 45242 (US)**

(74) Representative: **Vossius & Partner**
**Siebertstrasse 4 P.O. Box 86 07 67**
**D-8000 München 86 (DE)**

**EP 0 116 373 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Benzoxepine derivatives containing an amino group in 4-position of the oxepine ring are known from GB—A—1,211,258. These compounds are said to exhibit antidepressive and anticonvulsive actions.

This invention relates to 2*H*-[1]benzoxepino[5,4-*b*]-1,4-oxazine derivatives which may be substituted at the phenyl ring or at the amine nitrogen atom. More particularly, this invention relates to the *cis* and *trans* (4a,11b)-3,4,4a,5,6,11b-hexahydro-2*H*-[1]benzoxepino[5,4-*b*]-1,4-oxazine derivatives having the following general formula

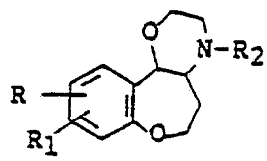

(I)

wherein R and $R_1$ are each hydrogen, hydroxy, $C_1$—$C_4$-alkoxy or when taken together are methylenedioxy, with the proviso that R and $R_1$ cannot both be hydrogen; $R_2$ is hydrogen, $C_1$—$C_4$-alkyl, phenyl-$C_1$—$C_4$-alkyl, $C_1$—$C_4$-alkoxy-$C_1$—$C_4$-alkyl, cyclopropylmethyl, 2-furanylmethyl and

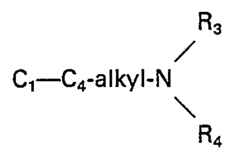

wherein $R_3$ and $R_4$ are each hydrogen or $C_1$—$C_4$-alkyl; and the pharmaceutical acceptable salts thereof. These derivatives possess useful antihypertensive activity. This invention further discloses a process whereby these derivatives can be conveniently prepared and in good yield.

Preferred are the compounds of the general formula I wherein $R_2$ is hydrogen and the compounds wherein R is $C_1$—$C_4$-alkoxy and $R_1$ is hydrogen.

As seen in general formula (I) above, the benzenoid moiety of the benzoxepino moiety can be mono or disubstituted as indicated by the symbols R and $R_1$. Due to the proviso limitation, *viz.*, the symbols R and $R_1$ cannot both be hydrogen at the same time. Thus, the phenyl ring of the benzoxepino moiety cannot be unsubstituted and must be either mono- or di-substituted. When substituted, the various substituents can be located at any one of the four positions on the benzenoid moiety, i.e., positions 8, 9, 10 or 11. The various substituents that are encompassed as being within the scope of the invention include the hydroxy, $C_1$—$C_4$-alkoxy and methylenedioxy groups either alone or in combination.

Examples for the $C_1$—$C_4$-alkoxy residues are the simple alkyl ethers wherein the alkyl group again contains from 1 to 4 straight or branched carbon atoms. Illustrative of such groups are the methyl, ethyl, propyl, isopropyl, butyl, isobutyl and *t*-butyl ethers.

Additionally, the ring nitrogen atom may be substituted or remain unsubstituted as indicated by the symbol $R_2$. The various substituents for the symbol $R_2$ that are included within the scope of this invention are the $C_1$—$C_4$-alkyl, phenyl-$C_1$—$C_4$-alkyl, $C_1$—$C_4$-alkoxy-$C_1$—$C_4$-alkyl, cyclopropylmethyl, 2-furanylmethyl, the

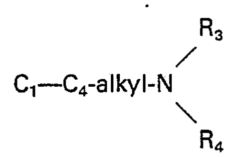

groups and those equivalents thereof that are known to the art. The symbols $R_3$ and $R_4$ represent either hydrogen or the $C_1$—$C_4$-alkyl radical and can be either N-$C_1$—$C_4$-alkyl or N,N-di-$C_1$—$C_4$-alkyl substituted. The N,N-di-$C_1$—$C_4$-alkyl group may be substituted with either the same $C_1$—$C_4$-alkyl group, or it may be substituted with a mixed $C_1$—$C_4$-alkyl group.

Examples for $C_1$—$C_4$-alkyl radicals are the methyl, ethyl, propyl, isopropyl, butyl, isobutyl or *t*-butyl radicals. The expression phenyl-$C_1$—$C_4$-alkyl relates to the 1,4-oxazine ring nitrogen being substituted by a $C_1$—$C_4$-alkyl group which terminates in a phenyl group, as for example, benzyl, phenethyl, phenpropyl, phenbutyl, etc.

The term $C_1$—$C_4$-alkoxy-$C_1$—$C_4$-alkyl represents those groups wherein the 1,4-oxazine ring nitrogen is substituted by a $C_1$—$C_4$-alkyl group which terminates in a $C_1$—$C_4$-alkyl ether, as for example, the 2-methoxyethyl, 3-ethoxypropyl and 4-propoxybutyl groups.

The term

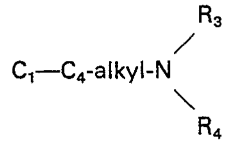

as used herein relates to those $C_1$—$C_4$-alkyl groups attached to the 1,4-oxazine ring nitrogen which terminate as either a primary, secondary or tertiary amine. Illustrative of such groups are the ethanamine, 2-propanamine, N-ethyl-ethanamine, N,N-dimethyl-ethanamine or N-methyl-N-propyl-3-propanamine groups.

The expression pharmaceutically acceptable salts refer to those non-toxic organic or inorganic acid addition salts which are equivalent to the above amines for the purposes of this invention. Illustrative inorganic acids which form suitable salts are hydrochloric, hydrobromic, sulfuric and phosphoric acid as well as acid metal salts such as sodium monohydrogen orthophosphate and potassium hydrogen sulfate. Illustrative organic acids which form suitable salts include the mono-, di- and tri-carboxylic acids, for example, acetic, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Either the mono- or the di-acid salts can be formed, and such salts can exist in either a hydrated or a substantially anhydrous form.

Illustrative compounds encompassed by the present invention are the *cis* and *trans* forms of:

(4a,11b)-10-ethoxy-3,4,4a,5,6,11b-hexahydro-4-propyl-2*H*-[1]benzoxepino[5,4-*b*]-1,4-oxazine;

(4a,11b)-8-butoxy-9-ethoxy-4-(3-ethoxypropyl)-3,4,4a,5,6,11b-hexahydro-2*H*-[1]benzoxepino[5,4-*b*]-1,4-oxazine;

(4a,11b)-3,4,4a,5,6,11b-hexahydro-9-methoxy-4-phenethyl-10-propoxy-2*H*-[1]benzoxepino[5,4-*b*]-1,4-oxazine;

(4a,11b)-4-(2-furanylmethyl)-3,4,4a,5,6,11b-hexahydro-9-hydroxy-2*H*-[1]benzoxepino[5,4-*b*]-1,4-oxazine;

(4a,11b)-4-dimethylaminoethyl-3,4,4a,5,6,11b-hexahydro-9-hydroxy-8-methoxy-2*H*-[1]benzoxepino[5,4-*b*]-1,4-oxazine;

(4a,11b)-4-cyclopropylmethyl-9,10,dihydroxy-3,4,4a,5,6,11b-hexahydro-2*H*-[1]benzoxepino[5,4-*b*]-1,4-oxazine;

(4a,11b)-3,4,4a,5,6,11b-hexahydro-8,9-methylenedioxy-4-(2-methylpropyl)-2*H*-[1]benzoxepino[5,4-*b*]-1,4-oxazine;

although the *trans* form compounds are preferred.

The novel (4a,11b)-3,4,4a,5,6,11b-hexahydro-2*H*-[1]benzoxepino[5,4-*b*]-1,4-oxazines of formula (I) can be readily prepared as illustrated in the following reaction scheme, wherein the symbols R, $R_1$ and $R_2$ are as defined above except that $R_2$ cannot be hydrogen.

*Reaction Scheme A:*

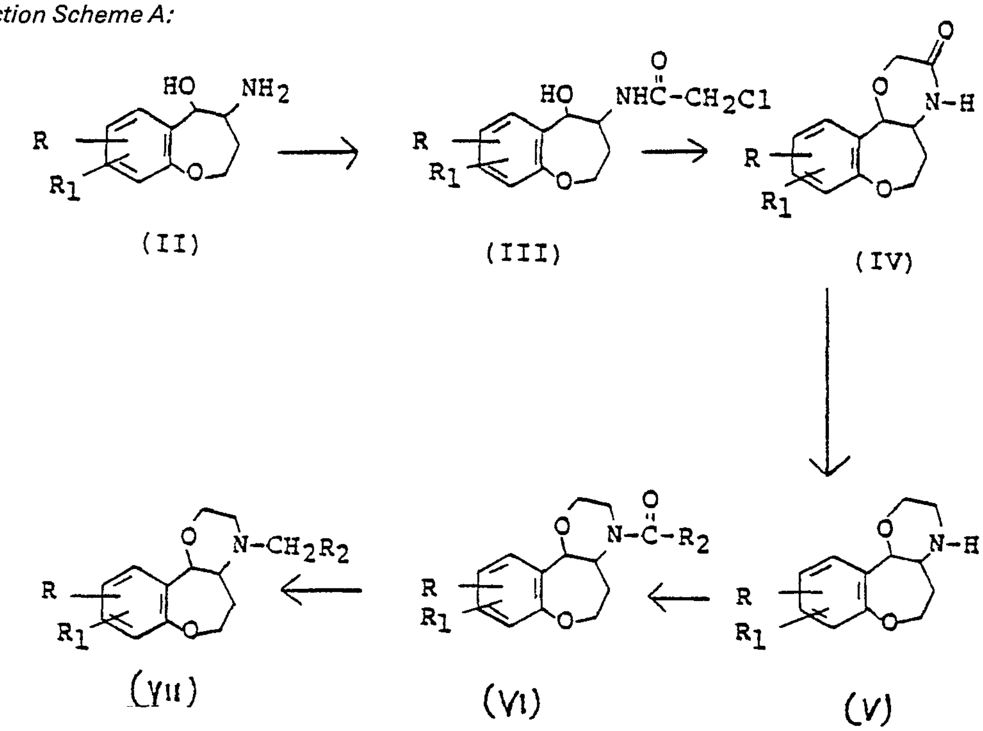

(II) (III) (IV)

(VII) (VI) (V)

In the formulae VI and VII $R_2'$ is defined as $R_2$ above, however, containing one methylene group less than $R_2$.

Thus, a substituted 4-amino-2,3,4,5-tetrahydro-1-benzoxepin-5-ol (II) can be acylated with chloroacetyl chloride in the presence of a trialkylamine, such as triethylamine to yield the corresponding substituted 2'-chloro-*N*-(2,3,4,5-tetrahydro-5-hydroxy-1-benzoxepin-4-yl)acetamide (III). The acylation can be conducted in an inert, halogenated solvent such as chloroform or methylene chloride at a temperature ranging from about 10° to about 30°C.

The chloroacetamido alcohol (III) can be cyclized with strong alkali in an aqueous alcohol solution. More particularly, cyclization can occur using a concentrated solution (50%) of sodium hydroxide in isopropanol or aqueous-isopropanol at room temperature for a period of from 8 to 24 hours.

The substituted (4a,11b)-4a,5,6,11b-tetrahydro-2*H*-[1]benzoxepino[5,4-*b*]-1,4-oxazine (IV) so obtained is reduced using a hydride reagent or diborane in an inert organic solvent. More particularly, lithium aluminum hydride can be favorably employed in a refluxing solvent, such as tetrahydrofuran or diethyl ether for a period of from about 3 to 12 hours. Where the symbol $R_2$ represents hydrogen in formula (I) above, the substituted (4a,11b)-3,4,4a,5,6,11b-hexahydro-2*H*-[1]benzoxepino[5,4-*b*]-1,4-oxazines (V) are obtained.

Alternatively, where the symbol $R_2$ is other than hydrogen or methyl in formula (I) above the resulting substituted (4a,11b)-3,4,4a,5,6,11b-hexahydro-2*H*-[1]-benzoxepino[5,4-*b*]-1,4-oxazines (V) are conveniently acylated with an acid chloride or an acid anhydride in the presence of a trialkylamine. Acylation can again be conducted in the presence of an inert halogenated solvent such as chloroform or methylene chloride to provide the corresponding substituted-*N*-acyl-(4a,11b)-3,4,4a,5,6,11b-hexahydro-2*H*-[1]-benzoxepino[5,4-*b*]-1,4-oxazines (VI).

Reduction of these *N*-acyl-2*H*-[1]benzoxepino[5,4-*b*]-1,4-oxazines (VI) using a hydride or diborane reagent provide the desired (4a,11b)-3,4,4a,5,6,11b-hexahydro-2*H*-[1]-benzoxepino[5,4-*b*]-1,4-oxazines (VII), wherein the symbol $R_2$ is other than hydrogen. More particularly, lithium aluminum hydride can be favorably employed in an inert, refluxing solvent, such as tetrahydrofuran or diethyl either for a period of from about 3 to 12 hours.

Alternatively, where the symbol $R_2$ represents the methyl group, it may be preferable to reduce the *N*-carboxylic acid ester in lieu of the *N*-acyl derivative in the last step of the reaction sequence. Thus, the substituted (4a,11b)-3,4,4a,5,6,11b-hexahydro-2*H*-[1]benzoxepino[5,4-*b*]-1,4-oxazines (V) can be reacted with an alkyl chloroformate in the presence of a trialkylamine and a solvent such as chloroform or methylene chloride to provide the corresponding substituted (4a,11b)-3,4,4a,5,6,11b-hexahydro-2*H*-[1]-benzoxepino[5,4-*b*]-1,4-oxazine derivatives (VIII) having the general formula

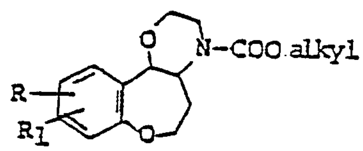

(VIII)

Reduction of this 4-carboalkoxy derivative with a hydride or diborane reagent, using essentially the same procedure as for the reduction of the *N*-acyl-(4a,11b)-3,4,4a,5,6,11b-hexahydro-2*H*-[1]-benzoxepino[5,4-*b*]-1,4-oxazine (VI), provides the desired 4-methyl derivative.

It is to be noted that the intermediate 4-amino-2,3,4,5-tetrahydrobenzoxepin-5-ol compounds (II) may exist in either their *cis*- or *trans*- geometric isomeric forms, each of which are enantiomeric mixtures which may be separated into individual enantiomers by methods known in the art such as by the formation of diastereomeric salts. Alternatively, each enantiomer can be synthesized from an optically pure starting material. All such isomers are embraced herein although throughout this specification it is preferred, from an end-use application of the compounds of this invention (I), to utilize the *trans* isomers.

The substituted 4-amino-2,3,4,5-tetrahydro-1-benzoxepin-5-ol compounds (II) useful as starting materials are either known compounds or compounds that can be readily prepared from known phenols or substituted phenols in accordance with the following reaction scheme:

*Reaction Scheme B:*

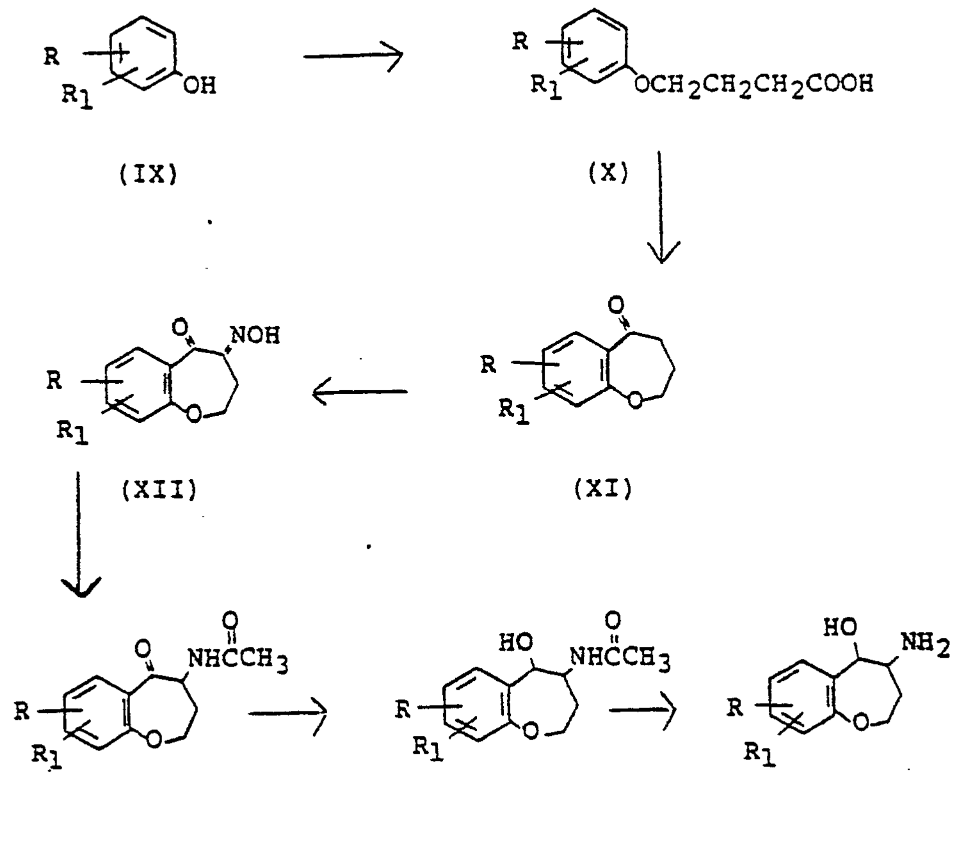

(IX) (X) (XII) (XI) (XIII) (XIV) (II)

Thus, phenol or a substituted phenol (IX) can be converted to the corresponding phenyloxybutyric acids (X) by heating the corresponding sodium phenolate with a slight excess of butyrolactone at a temperature of about 150—55°C. These phenyloxybutyric acids can be cyclized by heating with polyphosphoric acid at temperatures from about 55 to 100°C to yield the corresponding 2,3,4,5-tetrahydro-1-benzoxepin-5-ones (XI). The 2,3,4,5-tetrahydro-1-benzoxepin-5-ones so obtained (XI) can be dissolved in a solution of sodium ethoxide in ethanol and treated with isoamyl nitrite at ice-bath temperatures to form the 2,3,4,5-tetrahydro-1-benzoxepin-4,5-dione-4 oximes (XII).

The oximino ketones (XII) obtained in this manner can be reduced with zinc dust in an acetic acid/acetic anhydride mixture at temperatures of about 50—65°C to form the corresponding 2,3,4,5-tetrahydro-4-acetamido-1-benzoxepin-5-ones (XIII). Further reduction of these compounds by means of sodium borohydride in ethanol at temperatures ranging from about 10 to 30°C results in the formation of a mixture of the corresponding *cis*- and *trans*-2,3,4,5-tetrahydro-4-acetamido-1-benzoxepin-5-ols (XIV). Recrystallization of this mixture from a solvent such as ethyl acetate or acetonitrile results in the isolation of the desired isomers, preferably the *trans* isomer. Hydrolysis of these isomers (XIV) by means of a refluxing solution of aqueous sodium hydroxide in ethanol provides the desired *cis* or *trans* substituted 4-amino-2,3,4,5-tetrahydro-1-benzoxepin-5-ols (II), useful as starting materials for the preparation of the compounds of the present invention.

The compounds of this invention possess useful antihypertensive properties. In practicing this aspect of the invention an effective amount of one or more of the 2[H]-1-benzoxepino[5,4-*b*]-1,4-oxazines described herein are internally administered to a mammal in need thereof. In their end-use application as anti-hypertensive agents, the compounds most preferably are utilized in their *trans* isomeric form, although it is not essential that the administration of the preferred form be such that it be free of *any* of the *cis* form.

Administration can be carried out either via a parenteral route, such as by intravenous, intramuscular or intraperitoneal injection. Alternatively, the compounds disclosed herein can be introduced into the gastrointestinal tract via oral administration, there to be absorbed into the blood stream. Alternatively, these compounds can be introduced to mammals in need thereof via intratracheal administration, such as by inhalation of a solution of the drug in the form of a spray.

An effective antihypertensive amount is that amount of drug substance which is sufficient to lower or diminish the systemic arterial pressure in patients in need thereof. The particular amount of compound employed will vary widely depending upon various factors such as size, type, sex and age of the mammal

to be treated, in addition to the mode and frequency of administration, the compound utilized or the particular pharmaceutically acceptable salt employed as well as the degree of hypertension to be treated. In particular instances, the dosage to be administered can be determined via conventional range finding techniques, as for example, by monitoring the reduction in blood pressure at various dose levels.

The compounds herein described can be administered at dosages ranging from about 3 mg to about 3000 mg of a 2*H*-[1]benzoxepino[5,4-*b*]-1,4-oxazine derivative, which can be administered from one to four times daily. More particularly, oral dosages of from about 1 to about 20 milligrams per kilogram of animal body weight can be employed. Slightly lower parenteral dosages of from about 0.1 mg to about 10 milligrams per kilogram of animal body weight can be favorably employed.

It is generally desirable to administer the compounds of this invention in dosage unit form. A unit dosage may contain from about 1 to 500 mg of active ingredient, preferably from 5 to 150 mg of active ingredient, and can be taken one or more times per day. Dosage units suitable for oral administration include tablets, capsules, lozenges, elizirs, syrups and the like.

The active compound can be formulated via conventional procedures or as a timed release capsule or tablet formulations using techniques well known to those skilled in the art. Where rapid action is desired, the active ingredients may be formulated as injectable compositions, sprays or aerosols for inhalation therapy.

In the practice of this invention, the active ingredient is preferably formulated as compositions comprising from about 5 percent to about 90 percent by weight of the particular 2*H*-[1]-benzoxepino-[5,4-*b*]-1,4-oxazine, or a pharmaceutically acceptable salt thereof sought to be administered, in combination with a pharmaceutical carrier.

The term pharmaceutical carrier refers to those pharmaceutical excipients known to the art which are non-toxic and which are useful in the formulation of pharmaceutical compositions. Such compositions can be prepared via techniques known to those skilled in the art for the preparation of tablets, capsules, lozenges, troches, suppositories, elixirs, syrups, emulsions, dispersions, wettable and effervescent powders, sterile injectable compositions and solutions for sprays, and can contain suitable excipients known to be useful in the preparation of the particular type of composition desired. Suitable pharmaceutical carriers and formulation techniques are described in standard texts such as Remington's Pharmaceutical Sciences, 16th Edition (1980), Mack Publishing Company, Easton, Pennsylvania.

The following examples are provided to further illustrate the present invention but should not be construed as limiting the invention in any way.

### Example 1

Trans-(4a,11b)-3,4,4a,5,6,11b-Hexahydro-10-methoxy-2H-[1]benzoxepino[5,4-b]-1,4-oxazine Hydrochloride

A stirred suspension of 54 g (0.22 m) of 4-acetamido-7-methoxy-2,3-dihydroxybenzoxepin-5(1H)one in 600 ml of ethanol is cooled in an ice bath and 11 g of sodium borohydride is added (portionwise) under argon and the reaction mixture is stirred for an additional three hours. The solvent is evaporated, *in vacuo*, and the residue dissolved in one liter of water. The aqueous mixture is extracted with EtOAc (3 × 200 ml) and the combined extracts water washed (2 × 150 ml), dried ($MgSO_4$), filtered and evaporated to dryness *in vacuo* to yield a yellow solid which is digested with 1 liter EtOAc with extended heating. The remaining undissolved material is removed by filtration to yield 21.25 g of *trans*-4-amido-7-methoxy-2,3,4,5-tetrahydrobenzoxepin-5-ol. The mother liquor is cooled overnight and the formed solid removed by filtration. The resulting mother liquor is evaporated to dryness to give 20 g of *cis*-4-amido-7-methoxy-2,3,4,5-tetrahydrobenzoxepin-5-ol.

Separately, reflux 20 g of either of the so-obtained *cis* or *trans* amido alcohol, with 60 g of 50% aqueous sodium hydroxide and 150 ml of ethanol under argon for 5 hours. After cooling, each mixture is concentrated *in vacuo* and the residues poured into cold water (500 ml). On standing overnight, *trans*-4-amino-7-methoxy-2,3,4,5-tetrahydrobenzoxepin-5-ol precipitates. In the case of the *cis* compound the cold water solution is extracted with ethyl acetate (5 × 500 ml) followed by brine-washing, drying ($MgSO_4$) filtration, and evaporation of the combined extracts to yield the *cis*-4-amino-7-methoxy-2,3,4,5-tetrahydrobenzoxepin-5-ol.

A solution of 17 g of *trans*-4-amino-7-methoxy-2,3,4,5-tetrahydro-1-benzoxepin-5-ol dissolved in 700 ml of hydrocarbon-stabilized chloroform was treated with 16 ml of triethylamine at room temperature and 10 g of chloroacetyl chloride dissolved in 80 ml of chloroform slowly added thereto. The reaction mixture was allowed to stand at room temperature for 24 hours and the solid removed by filtration and washed with chloroform. The filtrate was washed twice with dilute hydrochloric acid solution, followed by a wash with water and dried over anhydrous magnesium sulfate. The filtrate was evaporated to dryness and triturated with ether to yield 18.8 gms of *trans*-2-chloro-*N*-(2,3,4,5-tetrahydro-5-hydroxy-7-methoxy-1-benzoxepin-4-yl)acetamide.

A solution of 18 g of this acetamide dissolved in 750 ml of isopropanol is treated with 9.5 ml of a 50% aqueous solution of sodium hydroxide. The mixture was stirred at room temperature overnight, concentrated *in vacuo* and diluted with approximately 1 liter of water. The insoluble *trans*-(4a,11b)-tetrahydro-10-methoxy-2*H*-[1]benzoxepino[5,4-*b*]-1,4-oxazine-3(4H)one, 13 g, was removed by filtration and dried. To a mixture of 3 g of lithium aluminum hydride in 500 ml of tetrahydrofuran is slowly added 12 g of the 1,4-oxazine-3(4H)one above. The reaction mixture is refluxed for a period of 5 hours, cooled in an

ice bath and treated with 13 ml of an aqueous 10% sodium hydroxide solution to decompose any excess hydride present. The mixture is stirred overnight, filtered and the filtrate concentrated *in vacuo*. The residue is dissolved in ether and treated with ethereal hydrogen chloride. The precipitated salt is filtered and recrystallized from methanol/acetonitrile to yield 10.8 grams of the title compound having a m.pt. of 260—2°C.

Following essentially the same procedure, but substituting *trans*-4-amino-9-methoxy-2,3,4,5-tetrahydro-1-benzoxepin-5-ol and *trans*-4-amino-8-methoxy-2,3,4,5-tetrahydro-1-benzoxepin-5-ol for the *trans*-4-amino-10-methoxy-2,3,4,5-tetrahydro-1-benzoxepin-5-ol above, results in the formation of *trans*-(4a,11b)-3,4,4a,5,6,11b-hexahydro-8-methoxy-2*H*-[1]benzoxepino[5,4-*b*]-1,4-oxazine hydrochloride, having a m.pt. of 254—6°C, and *trans*-(4a,11b)-3,4,4a,5,6,11b-hexahydro-9-methoxy-2*H*-[1]benzoxepino[5,4-*b*]-1,4-oxazine hydrochloride, having a m.pt. of 205—7°C.

## Example 2

Trans-(4a,11b)-3,4,4a,5,6,11b-Hexahydro-2H-[1]benzoxepino[5,4-b]-1,4-oxazine Hydrochloride

To a solution of 8.07 g of *trans*-4-amino-2,3,4,5-tetrahydro-1-benzoxepin-5-ol and 9 ml of triethylamine is 400 ml of chloroform is slowly added a solution of 5.58 g of chloroacetyl chloride in 50 ml of chloroform. The mixture is permitted to stand overnight at room temperature, washed with a dilute hydrochloric acid solution and filtered through a bed of magnesium sulfate. Evaporation of the filtrate and a recrystallization of the residue from toluene yielded 8.43 g of *trans*-2'-chloro-*N*-(2,3,4,5-tetrahydro-5-hydroxy-1-benzoxepin-4-yl)acetamide having a m.pt. of 141—4°C.

A solution of 8.3 g of the above amide dissolved in 460 ml of isopropanol is treated with 7.2 g of a 50% aqueous sodium hydroxide solution and the mixture stirred overnight at room temperature. The solvent is concentrated and diluted with water. The insoluble material which forms is removed by filtration and recrystallized from acetonitrile to yield 4.31 g of *trans*-(4a,11b)-4a,5,6,11b-tetrahydro-2*H*-[1]benzoxepino-[5,4-*b*]-1,4-oxazine-3(4H)one having a m.pt. of 246—8°C.

To a suspension of 1.5 g of lithium aluminum hydride in 100 ml of tetrahydrofuran is added, portionwise, 4.18 g of *trans*-(4a,11b)-4a,5,6,11b-tetrahydro-2H-[1]benzoxepino[5,4-*b*]-1,4-oxazine-3(4H)one. The mixture is refluxed for five hours, cooled in ice and excess hydride decomposed by the addition of 6 ml of a 10% sodium hydroxide solution. The resulting mixture is stirred overnight, the solids removed by filtration and the solvent removed by evaporation. The residue is dissolved in ether and treated with ethereal hydrogen chloride. The title compound which precipitates is recrystallized from a methanol/acetonitrile solution to yield 2.69 g of material having a m.pt. of 259—60°C.

## Example 3

Trans-(4a,11b)-3,4,4a,5,6,11b-Hexahydro-9-ethoxy-N,N-dimethyl-2H-[1]benzoxepino[5,4-b]-1,4-oxazine-4-ethanamine Maleate

A solution of 1.21 g of *trans*-(4a,11b)-3,4,4a,5,6,11b-hexahydro-9-ethoxy-2*H*-[1]benzoxepino-[5,4-*b*]-1,4-oxazine hydrochloride, prepared via an analogous procedure to the preceding Example, and 1.65 ml of triethylamine in 25 ml of methylene chloride is treated dropwise with a solution of 0.70 ml of chloroacetyl chloride dissolved in 10 ml of methylene chloride. The reaction mixture is stirred overnight at room temperature, washed with a dilute solution of hydrochloric acid, followed by a water wash, a wash of dilute sodium hydroxide solution and finally by a wash of a saturated solution of sodium chloride. The reaction mixture is dried over anhydrous magnesium sulfate and evaporated to dryness to obtain 1.44 g of *trans-N*-chloroacetyl-(4a,11b)-3,4,4a,5,6,11b-hexahydro-9-ethoxy-2*H*-[1]benzoxepino[5,4-*b*]-1,4-oxazine.

The *N*-chloroacetyl derivative above is dissolved in 50 ml of ethanol containing 1.5 grams of dimethylamine and the solution refluxed for approximately 4 hours. The reaction mixture is evaporated to dryness and the residue is taken up with ether. The ether solution is washed with water, followed by a saturated, aqueous brine solution and is dried over anhydrous magnesium sulfate. Treatment of the dried solution with ethereal hydrogen chloride results in the preparation of the *N*-dimethylaminoacetyl derivative as the hydrochloride salt.

To a stirred suspension of 1.0 g of lithium aluminum hydride in 50 ml of tetrahydrofuran is added the above hydrochloride salt of the *N*-dimethylaminoacetyl derivative in small portions. The mixture is refluxed for 2 hours under an inert atmosphere of argon, cooled in an ice bath, and cautiously decomposed by the addition of 1.5 ml of water. The mixture is stirred overnight, filtered and the filtrate is evaporated to dryness. The residue is dissolved in ether and treated with a solution of maleic acid in ether to precipitate the title compound as the maleate salt.

Following essentially the same procedure but substituting *trans*-(4a,11b)-3,4,4a,5,6,11b-hexahydro-9,10-methylenedioxy-2*H*-[1]benzoxepino-[5,4-*b*]-1,4-oxazine hydrochloride for the *trans*-(4a,11b)-3,4,4a,5,6,11b-hexahydro-9-ethoxy-2*H*-[1]benzoxepino-[5,4-*b*]-1,4-oxazine hydrochloride above results in the formation of *trans*-(4a,11b)-3,4,4a,5,6,11b-hexahydro-9,10-methylenedioxy-*N,N*-dimethyl-2*H*-[1]benzoxepino-[5,4-*b*]-1,4-oxazine-4-ethanamine maleate.

### Example 4

Trans-4-Ethyl-(4a,11b)-3,4,4a,5,6,11b-hexahydro-10-hydroxy-2H-[1]-benzoxepino[5,4-b]-1,4-oxazine Hydrochloride

A mixture of 3.47 g of *trans*-10-phenylmethoxy-(4a,11b)-3,4,4a,5,6,11b-hexahydro-2*H*-[1]benzoxepino-[5,4-*b*]-1,4-oxazine hydrochloride, 50 ml of triethylamine and 25 ml of methylene chloride is cooled in ice and 1.0 ml of acetyl chloride dissolved in 25 ml of methylene chloride is added dropwise thereto. The solution is stirred overnight at room temperature, washed with a dilute hydrochloric acid solution and dried over anhydrous magnesium sulfate. The drying agent is removed via filtration and the filtrate evaporated to yield *trans*-4-acetyl-10-phenylmethoxy-(4a,11b)-3,4,4a,5,6,11b-hexahydro-2*H*-[1]benzoxepino-[5,4-*b*]-1,4-oxazine as an oil.

The above 4-acetyl derivative is dissolved in 25 ml of tetrahydrofuran and added dropwise to an ice-cooled suspension of 1.0 g of lithium aluminum hydride in 50 ml of tetrahydrofuran. The mixture is refluxed for approximately 4 hours, cooled in ice and cautiously decomposed by means of 3.5 ml of a 10% sodium hydroxide solution. The reaction mixture is stirred overnight at room temperature, filtered and the filtrate evaporated to dryness *in vacuo*. The residue is dissolved in ether and treated with an ethereal hydrogen chloride solution to precipitate the *trans*-4-ethyl-10-phenylmethoxy-(4a,11b)-3,4,4a,5,6,11b-hexahydro-2*H*-[1]benzoxepino[5,4-*b*]-1,4-oxazine hydrochloride.

The 4-ethyl hydrochloride salt is dissolved in 50 ml of ethanol, shaken with hydrogen gas in a Parr shaker at 60 psi in the presence of 0.2 g of 10% palladium/charcoal until hydrogen uptake ceases. The catalyst is filtered, and the filtrate evaporated to dryness. The residue is triturated with ethyl acetate to yield the desired *trans*-4-ethyl-(4a,11b)-3,4,4a,5,6,11b-hexahydro-10-hydroxy-2*H*-[1]benzoxepino[5,4-*b*]-1,4-oxazine as the hydrochloride salt.

### Example 5

Trans-(4a,11b)-3,4,4a,5,6,11b-Hexahydro-10-methoxy-4-methyl-2H-[1]benzoxepino[5,4-b]-1,4-oxazine Maleate

A mixture of *trans*-(4a,11b)-3,4,4a,5,6,11b-hexahydro-10-methoxy-2*H*-[1]benzoxepino[5,4-*b*]-1,4-oxazine, 3.0 ml of triethylamine and 25 ml of methylene chloride is stirred and a solution of 1.0 ml of ethyl chloroformate dissolved in 25 ml of methylene chloride is added dropwise thereto. The mixture is stirred overnight, washed with a dilute hydrochloric acid solution, followed by a saturated sodium chloride solution wash, and dried over magnesium sulfate. The solvent is evaporated and the residue is dissolved in 25 ml of tetrahydrofuran.

This solution is added dropwise to a suspension of 1.0 g of lithium aluminum hydride in 50 ml of tetrahydrofuran. The resulting mixture is refluxed for 6 hours, cooled in an ice bath and excess hydride is decomposed via the cautious addition of 4.0 ml of a 10% aqueous solution of sodium hydroxide. The mixture is stirred overnight, filtered and the filtrate evaporated to dryness. The residue is dissolved in ether, treated with an ethereal solution of maleic acid to form the maleate salt. Recrystallization of this salt from a methanol/ethyl acetate mixture provides 1.97 g of the desired title compound.

Following essentially the same procedure but substituting *trans*-(4a,11b)-3,4,4a,5,6,11b-hexahydro-8-hydroxy-2*H*-[1]benzoxepino[5,4-*b*]-1,4-oxazine and *trans*-(4a,11b)-3,4,4a,5,6,11b-hexahydro-8,9-methylenedioxy-2*H*-[1]benzoxepino[5,4-*b*]-1,4-oxazine for the starting material above results in the formation of *trans*-(4a,11b)-3,4,4a,5,6,11b-hexahydro-8-hydroxy-2*H*-[1]benzoxepino[5,4-*b*]-1,4-oxazine maleate and *trans*-(4a,11b)-3,4,4a,5,6,11b-hexahydro-8,9-methylenedioxy-2*H*-[1]benzoxepino[5,4-*b*]-1,4-oxazine maleate, respectively.

Quite obviously, by starting with the *cis*-isomeric forms of the compounds of the foregoing examples and by following the same procedures of these examples there are produced the corresponding *cis*-isomers of the foregoing final compounds.

### Example 6

Preparation of a tablet formulation

One thousand tablets for oral use, each containing 125 mg of *trans*-(4a,11b)-3,4,4a,5,6,11b-hexahydro-10-methoxy-2*H*-[1]benzoxepino[5,4-*b*]-1,4-oxazine hydrochloride are prepared according to the following formulation:

| | Ingredients | Gm |
|---|---|---|
| (a) | *trans*-(4a,11b)-3,4,4a,5,6,11b-hexahydro-10-methoxy-2*H*-[1]-benzoxepino[5,4-*b*]-1,4-oxazine hydrochloride | 125 |
| (b) | Dicalcium phosphate | 150 |
| (c) | Methylcellulose, U.S.P. (15 cps) | 6.5 |
| (d) | Talc | 20 |
| (e) | Calcium stearate | 2.5 |

The *trans*-(4a,11b)-3,4,4a,5,6,11b-hexahydro-10-methoxy-2*H*-[1]benzoxepino[5,4-*b*]-1,4-oxazine hydrochloride and dicalcium phosphate are mixed well, granulated with a 7.5% aqueous solution of methylcellulose, passed through a No. 8 screen and carefully dried. The dried granules are passed through a No. 12 screen, blended with talc and calcium stearate and compressed into tablets.

Example 7

Preparation of a capsule formulation

One thousand two-piece hard gelatin capsules for oral use each containing 200 mg of *trans*-(4a,11b)-3,4,4a,5,6,11b-hexahydro-8-methoxy-2*H*-[1]benzoxepino[5,4-*b*]-1,4-oxazine hydrochloride are prepared from the following ingredients:

| | Ingredients | Gm |
|---|---|---|
| (a) | *trans*-(4a,11b)-3,4,4a,5,6,11b-hexahydro-8-methoxy-2*H*-[1]-benzoxepino[5,4-*b*]-1,4-oxazine hydrochloride | 200 |
| (b) | Lactose, U.S.P. | 100 |
| (c) | Starch U.S.P. | 10 |
| (d) | Talc, U.S.P. | 5 |
| (e) | Calcium stearate | 1 |

The finely powdered materials are mixed until uniformly dispersed and filled into hard shelled gelatin capsules of the appropriate size.

In a similar fashion one-piece soft gelatin capsules can be prepared in which the above formulation can be granulated, slugged or compressed directly into a rotary die or plate mold in which the capsule is formed. Alternatively, the above excipients may be omitted and the active ingredient dispensed as a powder directly into the capsule.

Example 8

The following example illustrates the anti-hypertensive activity obtained with the compounds of this invention.

Spontaneously hypertensive rats (males, 250—300 g), obtained from the Charles Rivers Breeding Laboratories, are divided into different treatment groups of 12 rats each. On separate days, one group of 12 rats is given 3, 6 or 10 mg/kg of the test compound, *trans*-(4a,11b)-3,4,4a,5,6,11b-hexahydro-10-methoxy-2*H*-[1]benzoxepino[5,4-*b*]-1,4-oxazine hydrochloride, by gavage and another group of 12 rats is given water (vehicle, 5 ml/kg).

Systolic arterial blood pressure is recorded from the tail of each rat using an occluding cuff and transducer. Systolic arterial blood pressure is recorded prior and subsequent to the administration of drug or vehicle at 1, 2, 3, 4, and 24 hours post treatment. The results are indicated as follows.

TABLE I

| Dosage of test compound (mg/kg) | Change in systolic blood pressure from Control post treatment (mm Hg) | | | | |
|---|---|---|---|---|---|
| | 1Hr. | 2 Hr. | 3 Hr. | 4 Hr. | 5 Hr. |
| 3 | −21* | −20* | −22* | −19* | −15* |
| 6 | −33* | −26* | −30* | −35* | −7 |
| 10 | −43* | −51* | −46* | −42* | −21* |
| Control (water, 5 mg/kg) | −4 | −6 | −5 | −8 | +2 |

* Statistically significant ($p<0.05$)

As can be seen, the test compound produced a dose related decrease in systolic arterial blood pressure that is statistically significant. The duration of the antihypertensive effect is between 4 and 24 hours for the 6 mg/kg treatment group, and more than 24 hours in the 3 and 10 mg/kg group.

As noted above, in their end-use application as anti-hypertensive agents, it is always preferred to utilize the *trans*-form of the compounds of this invention (I), although in such preference it is not absolutely essential that the *trans* compound be absolutely free from the *cis* isomer.

**Claims for the Contracting States: BE CH DE FR GB IT LI LU NL SE**

1. A (4a,11b)-3,4,4a,5,6,11b-hexahydro-2*H*-[1]benzoxepino-[5,4-*b*]-1,4-oxazine having the general formula I

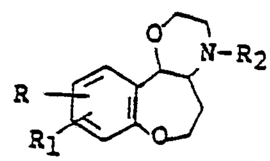

(I)

including their *cis* and *trans* isomers, and the enantiomeric forms thereof, wherein R and $R_1$ are each hydrogen, hydroxy, $C_1$—$C_4$-alkoxy or when taken together are methylenedioxy, with the proviso that R and $R_1$ cannot both be hydrogen; $R_2$ is hydrogen, $C_1$—$C_4$-alkyl, phenyl-$C_1$—$C_4$-alkyl, $C_1$—$C_4$-alkoxy-$C_1$—$C_4$-alkyl, cyclopropylmethyl, 2-furanylmethyl and

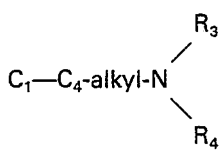

wherein $R_3$ and $R_4$ are each hydrogen or $C_1$—$C_4$-alkyl; and the pharmaceutically acceptable salts thereof.

2. A compound according to claim 1 wherein $R_2$ is hydrogen.

3. A compound according to claim 1 wherein R is $C_1$—$C_4$-alkoxy and $R_1$ is hydrogen.

4. A compound according to claim 1 which is *trans*-(4a,11b)-3,4,4a,5,6,11b-hexahydro-10-methoxy-2*H*-[1]benzoxepino[5,4-*b*]-1,4-oxazine.

5. A compound according to claim 1 which is *trans*-(4a,11b)-3,4,4a,5,6,11b-hexahydro-9-methoxy-2*H*-[1]benzoxepino[5,4-*b*]-1,4-oxazine.

6. A compound according to claim 1 which is *trans*-(4a,11b)-3,4,4a,5,6,11b-hexahydro-8-methoxy-2*H*-[1]benzoxepino[5,4-b]-1,4-oxazine.

7. A process for the preparation of a compound having the general formula I according to claim 1

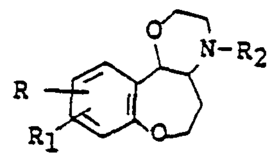

(I)

which comprises:

(1) reacting a 4-amino-2,3,4,5-tetrahydro-1-benzoxepin-5-ol having the general formula II

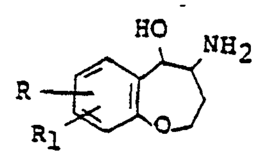

(II)

with chloroacetyl chloride in the presence of a trialkylamine to prepare a 2'-chloro-*N*-(2,3,4,5-tetrahydro-5-hydroxy-1-benzoxepin-4-yl)acetamide; cyclizing said acetamide with sodium hydroxide to prepare a (4a,11b)-4a,5,6,11b-tetrahydro-2*H*-[1]benzoxepino[5,4-*b*]-1,4-oxazine-3(4H)one having the general formula IV;

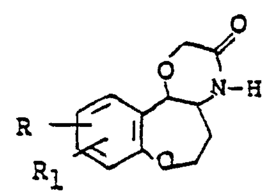

(IV)

and reducing said 1,4-oxazine-3(4H)one using a hydride reagent or diborane in an inert organic solvent to prepare a (4a,11b)-3,4,4a,5,6,11b-hexahydro-2*H*-[1]benzoxepino[5,4-*b*]-1,4-oxazine having the general formula V (a compound of the general formula I wherein $R_2$ is hydrogen);

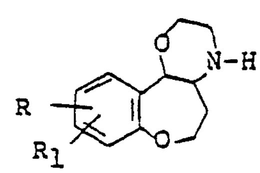

(V)

(2) for preparing a compound of the general formula I wherein $R_2$ is other than hydrogen or methyl, acylating said benzoxepino[5,4-*b*]-1,4-oxazine of formula V with an acid chloride or an acid anhydride in the presence of a trialkylamine to prepare an *N*-acyl-(4a,11b)-3,4,4a,5,6,11b-hexahydro-2*H*-[1]benzoxepino[5,4-*b*]-1,4-oxazine having the general formula VI;

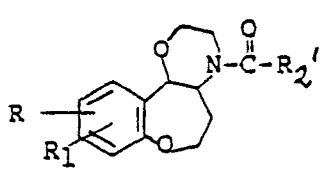

(VI)

wherein $R_2'$ is defined as $R_2$ above however containing one methylene group less than $R_2$; and reducing said *N*-acyl-benzoxepino[5,4-*b*]-1,4-oxazine using a hydride reagent or diborane in an inert organic solvent and recovering the desired (4a,11b)-3,4,4a,5,6,11b-hexahydro-2*H*-[1]-benzoxepino[5,4-*b*]-1,4-oxazine therefrom, or

(3) for preparing a compound of the general formula I, wherein $R_2$ is methyl, reacting said benzoxepino[5,4-b]-1,4-oxazine of formula V with an alkyl chloroformate in the presence of a trialkylamine to prepare a (4a,11b)-3,4,4a,5,6,11b-hexahydro-4-carboalkoxy-2H-[1]-benzoxepino[5,4-*b*]-1,4-oxazine derivative of the general formula VIII

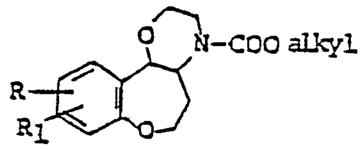

(VIII)

and reducing said benzoxepino[5,4-b]-1,4-oxazine derivative using a hydride reagent or diborane in an inert organic solvent and recovering the desired 4-methyl derivative.

8. Pharmaceutical composition comprising a compound as set forth in claim 1.

**Claims for the Contracting State: AT**

1. A process for the preparation of a (4a,11b)-3,4,4a,5,6,11b-hexahydro-2*H*-[1]benzoxepino-[5,4-*b*]-1,4-oxazine having the general formula I

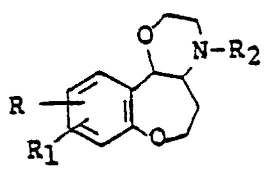

(I)

including their *cis* and *trans* isomers, and the enantiomeric forms thereof, wherein R and $R_1$ are each hydrogen, hydroxy, $C_1$—$C_4$-alkoxy or when taken together are methylenedioxy, with the proviso that R and $R_1$ cannot both be hydrogen; $R_2$ is hydrogen, $C_1$—$C_4$-alkyl, phenyl-$C_1$—$C_4$-alkyl, $C_1$—$C_4$-alkoxy-$C_1$—$C_4$-alkyl, cyclopropylmethyl, 2-furanylmethyl and

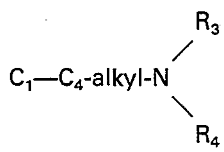

wherein $R_3$ and $R_4$ are each hydrogen or $C_1$—$C_4$-alkyl; and the pharmaceutically acceptable salts thereof, which comprises:

(1) reacting a 4-amino-2,3,4,5-tetrahydro-1-benzoxepin-5-ol having the general formula II

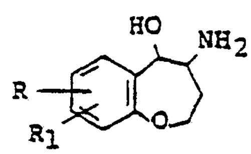

(II)

with chloroacetyl chloride in the presence of a trialkylamine to prepare a 2′-chloro-*N*-(2,3,4,5-tetrahydro-5-hydroxy-1-benzoxepin-4-yl)acetamide; cyclizing said acetamide with sodium hydroxide to prepare a (4a,11b)-4a,5,6,11b-tetrahydro-2*H*-[1]benzoxepino[5,4-*b*]-1,4-oxazine-3(4H)one having the general formula IV;

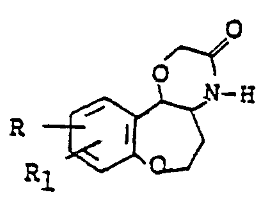

(IV)

and reducing said 1,4-oxazine-3(4H)one using a hydride reagent or diborane in an inert organic solvent to prepare a (4a,11b)-3,4,4a,5,6,11b-hexahydro-2*H*-[1]benzoxepino[5,4-*b*]-1,4-oxazine having the general formula V (a compound of the general formula I wherein $R_2$ is hydrogen);

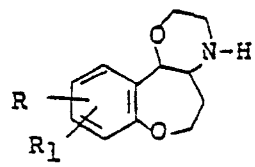

(V)

(2) for preparing a compound of the general formula I wherein $R_2$ is other than hydrogen or methyl, acylating said benzoxepino[5,4-*b*]-1,4-oxazine of formula V with an acid chloride or an acid anhydride in the presence of a trialkylamine to prepare an *N*-acyl-(4a,11b)-3,4,4a,5,6,11b-hexahydro-2*H*-[1]benzoxepino[5,4-*b*]-1,4-oxazine having the general formula VI;

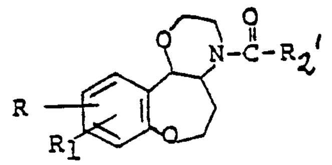

(VI)

wherein $R_2'$ is defined as $R_2$ above however containing one methylene group less than $R_2$; and reducing said *N*-acyl-benzoxepino[5,4-*b*]-1,4-oxazine using a hydride reagent or diborane in an inert organic solvent and recovering the desired (4a,11b)-3,4,4a,5,6,11b-hexahydro-2*H*-[1]-benzoxepino[5,4-*b*]-1,4-oxazine therefrom, or

(3) for preparing a compound of the general formula I, wherein $R_2$ is methyl, reacting said benzoxepino[5,4-b]-1,4-oxazine of formula V with an alkyl chloroformate in the presence of a trialkylamine to prepare a (4a,11b)-3,4,4a,5,6,11b-hexahydro-4-carboalkoxy-2H-[1]-benzoxepino[5,4-*b*]-1,4-oxazine derivative of the general formula VIII

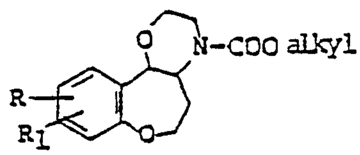

(VIII)

and reducing said benzoxepino[5,4-b]-1,4-oxazine derivative using a hydride reagent or diborane in an inert organic solvent and recovering the desired 4-methyl derivative.

2. The process according to claim 1 for the production of *trans*-(4a,11b)-3,4,4a,5,6,11b-hexahydro-10-methoxy-2*H*-[1]benzoxepino[5,4-*b*]-1,4-oxazine.

3. The process according to claim 1 for the production of *trans*-(4a,11b)-3,4,4a,5,6,11b-hexahydro-9-methoxy-2*H*-[1]benzoxepino[5,4-*b*]-1,4-oxazine.

4. The process according to claim 1 for the production of *trans*-(4a,11b)-3,4,4a,5,6,11b-hexahydro-8-methoxy-2*H*-[1]benzoxepino[5,4-b]-1,4-oxazine.

**Patentansprüche für die Vertragsstaaten: BE CH DE FR GB IT LI LU NL SE**

1. Ein (4a,11b)-3,4,4a,5,6,11b-Hexahydro-2*H*-[1]benzoxepino[5,4-b]-1,4-oxazin der allgemeinen Formel I

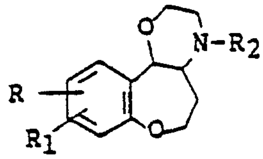

(I)

einschließlich ihrer cis- und trans-Isomeren und der enantiomeren Formen davon, in der R und $R_1$ jeweils ein Wasserstoffatom, eine Hydroxylgruppe oder einen $C_1$—$C_4$-Alkoxyrest bedeuten oder zusammengenommen eine Methylendioxygruppe darstellen, mit der Maßgabe, daß R und $R_1$ nicht beide Wasserstoffatome bedeuten können; $R_2$ ein Wasserstoffatom, einen $C_1$—$C_4$-Alkyl-, Phenyl-$C_1$—$C_4$-Alkyl-, $C_1$—$C_4$-Alkoxy-$C_1$—$C_4$-alkyl-, Cyclopropylmethyl- oder 2-Furanylmethylrest oder einen Rest

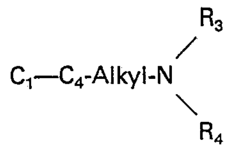

darstellt, wobei $R_3$ und $R_4$ jeweils Wasserstoffatome oder $C_1$—$C_4$-Alkylreste bedeuten; und die pharmazeutisch verträglichen Salze davon.

2. Eine Verbindung nach Anspruch 1, in der $R_2$ ein Wasserstoffatom ist.

3. Eine Verbindung nach Anspruch 1, in der R einen $C_1$—$C_4$-Alkoxyrest bedeutet und $R_1$ ein Wasserstoffatom ist.

4. Eine Verbindung nach Anspruch 1, nämlich trans-(4a,11b)-3,4,4a,5,6,11b-Hexahydro-10-methoxy-2H-[1]benzoxepino[5,4-b]-1,4-oxazin.

5. Eine Verbindung nach Anspruch 1, nämlich trans-(4a,11b)-3,4,4a,5,6,11b-Hexahydro-9-methoxy-2H-[1]benzoxepino[5,4-b]-1,4-oxazin.

6. Eine Verbindung nach Anspruch 1, nämlich trans-(4a,11b)-3,4,4a,5,6,11b-Hexahydro-8-methoxy-2H-[1]-benzoxepino[5,4-b]-1,4-oxazin.

7. Verfahren zur Herstellung einer Verbindung der allgemeinen Formel I nach Anspruch 1

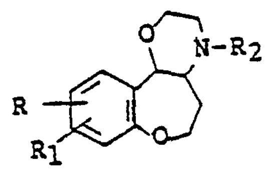

(I)

durch:

(1) Umsetzung eines 4-Amino-2,3,4,5-tetrahydro-1-benzoxepin-5-ols der allgemeinen Formel II

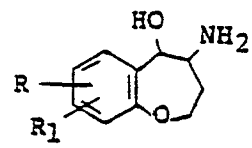

(II)

mit Chloracetylchlorid in Gegenwart eines Trialkylamins zur Herstellung eines 2'-Chlor-N-(2,3,4,5-tetrahydro-5-hydroxy-1-benzoxepin-4-yl)-acetamids; Cyclisierung des Acetamids mit Natriumhydroxid zur Herstellung eines (4a,11b)-4a,5,6,11b-Tetrahydro-2H-[1]benzoxepino[5,4-b]-1,4-oxazin-3(4H)ons der allgemeinen Formel IV;

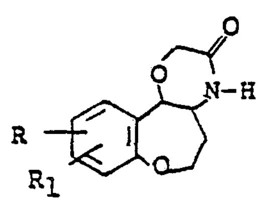

(IV)

und Reduktion des 1,4-Oxazin-3(4H)ons mit einem Hydrid-Reagens oder Diboran in einem inerten organischen Lösungsmittel zur Herstellung eines (4a,11b)-3,4,4a,5,6,11b-Hexahydro-2H-[1]benzoxepino[5,4-b]-1,4-oxazins der allgemeinen Formel V (eine Verbindung der allgemeinen Formel I, in der $R_2$ ein Wasserstoffatom ist);

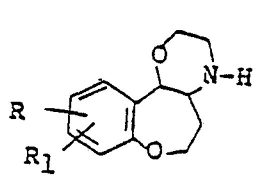

(V)

(2) zur Herstellung einer Verbindung der allgemeinen Formel I, in der $R_2$ eine andere Bedeutung als Wasserstoffatom oder Methylgruppe hat, Acylierung des genannten Benzoxepino[5,4-b]-1,4-oxazins der Formel V mit einem Säurechlorid oder einem Säureanhydrid in Gegenwart eines Trialkylamins zur

Herstellung eines N-Acyl-(4a,11b)-3,4,4a,5,6,11b-hexahydro-2H-[1]benzoxepino[5,4-b]-1,4-oxazins der allgemeinen Formel VI

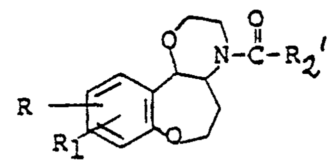

(VI)

in der $R_2'$ wie $R_2$ vorstehend definiert ist, jedoch eine Methylengruppe weniger als $R_2$ enthält; und Reduktion des N-Acyl-benzoxepino[5,4-b]-1,4-oxazins mit einem Hydrid-Reagens oder Diboran in einem inerten organischen Lösungsmittel und Gewinnung des gewünschten (4a,11b)-3,4,4a,5,6,11b-Hexahydro-2H-[1]-benzoxepino[5,4-b]-1,4-oxazins daraus, oder

(3) zur Herstellung einer Verbindung der allgemeinen Formel I, in der $R_2$ eine Methylgruppe bedeutet, Umsetzung des Benzoxepino[5,4-b]-1,4-oxazins der Formel V mit einem Chlorameisensäurealkylester in Gegenwart eines Trialkylamins zur Herstellung eines (4a,11b)-3,4,4a,5,6,11b-Hexahydro-4-carboalkoxy-2H-[1]-benzoxepino[5,4-b]-1,4-oxazin-Derivates der allgemeinen Formel VIII

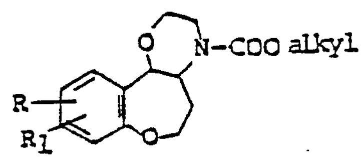

(VIII)

und Reduktion des Benzoxepino[5,4-b]-1,4-oxazin-Derivates mit einem Hydrid-Reagens oder Diboran in einem inerten organischen Lösungsmittel und Gewinnung des gewünschten 4-Methyl-Derivates.

8. Arzneimittel, umfassend eine Verbindung nach Anspruch 1.

**Patentansprüche für den Vertragsstaat: AT**

1. Ein Verfahren zur Herstellung eines (4a,11b)-3,4,4a,5,6,11b-Hexahydro-2H-[1]benzoxepino[5,4-b]-1,4-oxazins der allgemeinen Formel I

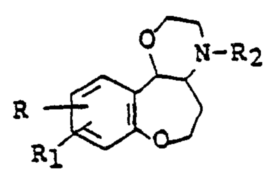

(I)

einschließlich ihrer cis- und trans-Isomeren und der enantiomeren Formen davon, in der R und $R_1$ jeweils ein Wasserstoffatom, eine Hydroxylgruppe oder einen $C_1$—$C_4$-Alkoxyrest bedeuten oder zusammengenommen eine Methylendioxygruppe darstellen, mit der Maßgabe, daß R und $R_1$ nicht beide Wasserstoffatome bedeuten können; $R_2$ ein Wasserstoffatom, einen $C_1$—$C_4$-Alkyl-, Phenyl-$C_1$—$C_4$-Alkyl-, $C_1$—$C_4$-Alkoxy-$C_1$—$C_4$-alkyl-, Cyclopropylmethyl- oder 2-Furanylmethylrest oder einen Rest

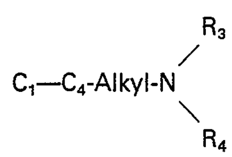

darstellt, wobei $R_3$ und $R_4$ jeweils Wasserstoffatome oder $C_1$—$C_4$-Alkylreste bedeuten; und die pharmazeutisch verträglichen Salze davon, durch:

(1) Umsetzung eines 4-Amino-2,3,4,5-tetrahydro-1-benzoxepin-5-ols der allgemeinen Formel II

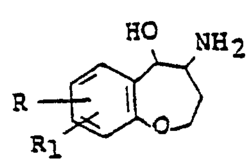

(II)

mit Chloracetylchlorid in Gegenwart eines Trialkylamins zur Herstellung eines 2'-Chlor-N-(2,3,4,5-tetrahydro-5-hydroxy-1-benzoxepin-4-yl)-acetamids; Cyclisierung des Acetamids mit Natriumhydroxid zur Herstellung eines (4a,11b)-4a,5,6,11b-Tetrahydro-2H[1]benzoxepino[5,4-b]-1,4-oxazin-3(4H)ons der allgemeinen Formel IV;

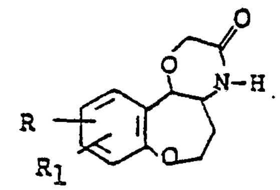

(IV)

und Reduktion des 1,4-Oxazin-3(4H)ons mit einem Hydrid-Reagens oder Diboran in einem inerten organischen Lösungsmittel zur Herstellung eines (4a,11b)-3,4,4a,5,6,11b-Hexahydro-2H-[1]benzoxepino[5,4-b]-1,4-oxazins der allgemeinen Formel V (eine Verbindung der allgemeinen Formel I, in der $R_2$ ein Wasserstoffatom ist);

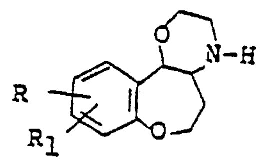

(V)

(2) zur Herstellung einer Verbindung der allgemeinen Formel I, in der $R_2$ eine andere Bedeutung als Wasserstoffatom oder Methylgruppe hat, Acylierung des genannten Benzoxepino[5,4-b]-1,4-oxazins der Formel V mit einem Säurechlorid oder einem Säureanhydrid in Gegenwart eines Trialkylamins zur Herstellung eines N-Acyl-(4a,11b)-3,4,4a,5,6,11b-hexahydro-2H-[1]benzoxepino[5,4-b]-1,4-oxazins der allgemeinen Formel VI

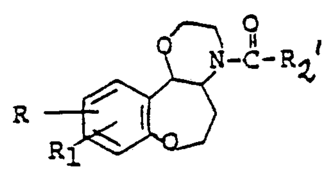

(VI)

in der $R_2'$ wie $R_2$ vorstehend definiert ist, jedoch eine Methylengruppe weniger als $R_2$ enthält; und Reduktion des N-Acyl-benzoxepino[5,4-b]-1,4-oxazins mit einem Hydrid-Reagens oder Diboran in einem inerten organischen Lösungsmittel und Gewinnung des gewünschten (4a,11b)-3,4,4a,5,6,11b-Hexahydro-2H-[1]-benzoxepino[5,4-b]-1,4-oxazins daraus, oder

(3) zur Herstellung einer Verbindung der allgemeinen Formel I, in der $R_2$ eine Methylgruppe bedeutet, Umsetzung des Benzoxepino[5,4-b]-1,4-oxazins der Formel V mit einem Chlorameisensäurealkylester in Gegenwart eines Trialkylamins zur Herstellung eines (4a,11b)-3,4,4a,5,6,11b-Hexahydro-4-carboalkoxy-2H-[1]-benzoxepino[5,4-b]-1,4-oxazin-Derivates der allgemeinen Formel VIII

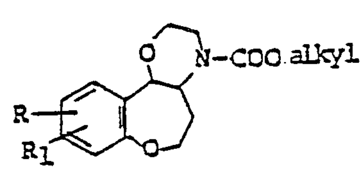

(VIII)

und Reduktion des Benzoxepino[5,4-b]-1,4-oxazin-Derivates mit einem Hydrid-Reagens oder Diboran in einem inerten organischen Lösungsmittel und Gewinnung des gewünschten 4-Methyl-Derivates.

2. Verfahren nach Anspruch 1 zur Herstellung von trans-(4a,11b)-3,4,4a,5,6,11b-Hexahydro-10-methoxy-2H-[1]benzoxepino[5,4-b]-1,4-oxazin.

3. Verfahren nach Anspruch 1 zur Herstellung von trans-(4a,11b)-3,4,4a,5,6,11b-Hexahydro-9-methoxy-2H-[1]benzoxepino[5,4-b]-1,4-oxazin.

4. Verfahren nach Anspruch 1 zur Herstellung von trans-(4a,11b)-3,4,4a,5,6,11b-Hexahydro-8-methoxy-2H-[1]benzoxepino[5,4-b]-1,4-oxazin.

**Revendications pour les Etats contractants: BE CH DE FR GB IT LI LU NL SE**

1. Une (4a,11b)-3,4,4a,5,6,11b-hexahydro-2*H*-[1]benzoxépino-[5,4-*b*]-1,4-oxazine ayant la formule générale (I)

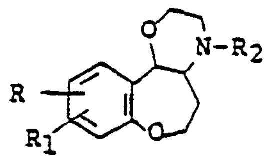

(I)

ce qui comprend leurs isomères *cis* et *trans*, et leurs formes énantiomères, dans laquelle R et $R_1$ représentent chacun un atome d'hydrogène, un groupe hydroxy, alcoxy en $C_1$—$C_4$ ou, quand ils sont pris ensemble, ils forment un groupe méthylènedioxy, à la condition que R et $R_1$ ne peuvent pas simultanément être chacun un atome d'hydrogène; $R_2$ représente un atome d'hydrogène, un groupe alkyle en $C_1$—$C_4$, phényl-alkyle en $C_1$—$C_4$, alcoxy(en $C_1$—$C_4$)alkyle en $C_1$—$C_4$, cyclopropylméthyle, 2-furanylméthyle et

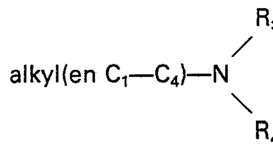

,

où $R_3$ et $R_4$ représentent chacun un atome d'hydrogène ou un groupe alkyle en $C_1$—$C_4$, et leurs sels pharmaceutiquement acceptables.

2. Composé selon la revendication 1, dans lequel $R_2$ représente un atome d'hydrogène.

3. Composé selon la revendication 1, dans lequel R représente un groupe alcoxy en $C_1$—$C_4$ et $R_1$ est un atome d'hydrogène.

4. Composé selon la revendication 1, qui est la *trans*-(4a,11b)-3,4,4a,5,6,11b-hexahydro-10-méthoxy-2*H*-[1]benzoxépino[5,4-*b*]-1,4-oxazine.

5. Composé selon la revendication 1, qui est la *trans*-(4a,11b)-3,4,4a,5,6,11b-hexahydro-9-méthoxy-2*H*-[1]benzoxépino[5,4-*b*]-1,4-oxazine.

6. Composé selon la revendication 1, qui est la *trans*-(4a,11b)-3,4,4a,5,6,11b-hexahydro-8-méthoxy-2*H*-[1]benzoxépino[5,4-*b*]-1,4-oxazine.

7. Procédé pour la préparation d'un composé ayant la formule générale (I) selon la revendication 1

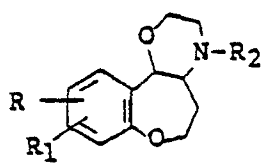

(I)

qui comprend:

(1) la réaction d'un 4-amino-2,3,4,5-tétrahydro-1-benzoxépine-5-ol ayant la formule générale (II)

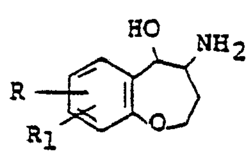

(II)

avec le chlorure de chloroacétyle en présence d'une trialkylamine pour préparer un 2'-chloro-*N*-(2,3,4,5-tétrahydro-5-hydroxy-1-benzoxépine-4-yl)acétamide; la cyclisation dudit acétamide par de l'hydroxyde de sodium pour préparer une (4a,11b)-4a,5,6,11b-tétrahydro-2*H*-[1]benzoxépino[5,4-*b*]-1,4-oxazine-3(4H)one ayant la formule générale (IV)

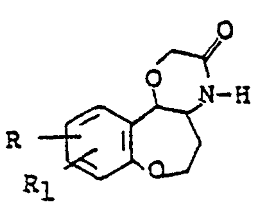

(IV)

et la réduction de ladite 1,4-oxazine-3(4H)one, en utilisant un réactif du type hydrure ou du diborane dans un solvant organique inerte pour préparer une (4a,11b)-3,4,4a,5,6,11b-hexahydro-2*H*-[1]benzoxépino[5,4-*b*]-1,4-oxazine ayant la formule générale (V) (composé de formule générale I dans laquelle $R_2$ représente un atome d'hydrogène);

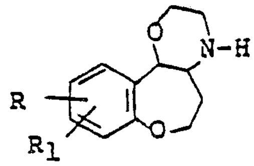

(V)

(2) pour préparer un composé de formule générale (I), dans laquelle $R_2$ représente autre chose qu'un atome d'hydrogène ou un groupe méthyle, l'acylation de ladite benzoxépino-[5,4-*b*]-1,4-oxazine de formule (V) par un chlorure d'acide ou un anhydride d'acide en présence d'une trialkylamine pour préparer une *N*-acyl-(4a,11b)-3,4,4a,5,6,11b-hexahydro-2*H*-[1]benzoxépino-[5,4-*b*]-1,4-oxazine ayant la formule générale (VI)

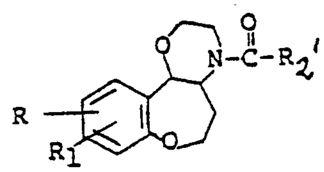

(VI)

dans laquelle $R_2'$ est défini comme $R_2$ ci-dessus, mais contient un groupe méthylène de moins que $R_2$; et la réduction de ladite *N*-acyl-benzoxépino[5,4-*b*]-1,4-oxazine en utilisant un hydrure ou du diborane dans un solvant organique inerte et la récupération de la (4a,11b)-3,4,4a,5,6,11b-hexahydro-2*H*-[1]-benzoxépino[5,4-*b*]-1,4-oxazine voulue, ou

(3) pour préparer un composé de formule générale (I), dans laquelle $R_2$ représente un groupe méthyle, la réaction de ladite benzoxépino[5,4-b]-1,4-oxazine de formule (V) avec un chloroformiate d'alkyle en présence d'une trialkylamine pour préparer un dérivé de type (4a,11b)-3,4,4a,5,6,11b-hexahydro-4-carbo-alcoxy-2*H*-[1]-benzoxépino[5,4-*b*]-1,4-oxazine de formule générale VIII

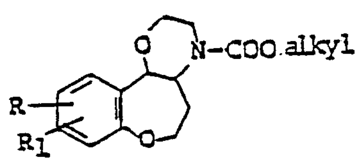

(VIII)

et la réduction dudit dérivé de benzoxépino[5,4-*b*]-1,4-oxazine à l'aide d'un hydrure ou de diborane dans un solvant organique inerte et la récupération du dérivé de 4-méthyle voulu.

8. Composition pharmaceutique comprenant un composé tel que présenté à la revendication 1.

**Revendications pour l'Etat contractant: AT**

1. Procédé pour la préparation d'une (4a,11b)-3,4,4a,5,6,11b-hexahydro-2*H*-[1]benzoxépino-[5,4-*b*]-1,4-oxazine ayant la formule générale (I)

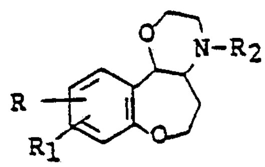

(I)

ce qui comprend leur isomères *cis* et *trans*, et leurs formes énantiomères, formule dans laquelle R et $R_1$ représentent chacun un atome d'hydrogène, un groupe hydroxy, alcoxy en $C_1$—$C_4$ ou, quand ils sont pris ensemble, ils forment un groupe méthylène dioxy, à la condition que R et $R_1$ ne puissent représenter simultanément chacun un atome d'hydrogène; $R_2$ représente un atome d'hydrogène, un groupe alkyle en $C_1$—$C_4$, phényl-alkyle en $C_1$—$C_4$, alcoxy(en $C_1$—$C_4$)alkyle en $C_1$—$C_4$, cyclopropylméthyle, 2-furanylméthyle et

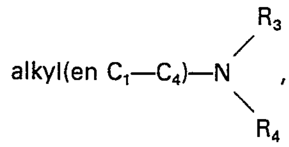

où $R_3$ et $R_4$ représentent chacun un atome d'hydrogène ou un groupe alkyle en $C_1$—$C_4$, et leurs sels pharmaceutiquement acceptables, qui comprend:

(1) la réaction d'un 4-amino-2,3,4,5-tétrahydro-1-benzoxépine-5-ol ayant la formule générale (II)

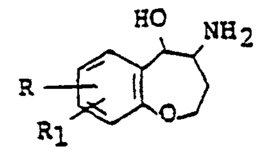

(II)

avec du chlorure de chloroacétyle en présence d'une trialkylamine pour préparer un 2'-chloro-*N*-(2,3,4,5-tétrahydro-5-hydroxy-1-benzoxépine-4-yl)acétamide; la cyclisation dudit acétamide avec de l'hydroxyde de sodium pour préparer une (4a,11b)-4a,5,6,11b-tétrahydro-2*H*-[1]benzoxépino[5,4-*b*]-1,4-oxazine-3(4H)one ayant la formule générale (IV)

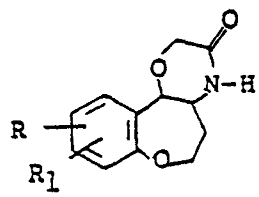

(IV)

et la réduction de ladite 1,4-oxazine-3(4H)one en utilisant un hydrure ou du diborane dans un solvant organique inerte pour préparer une (4a,11b)-3,4,4a,5,6,11b-hexahydro-2*H*-[1]benzoxépino[5,4-*b*]-1,4-oxazine ayant la formule générale (V) (composé de formule générale I dans laquelle $R_2$ représente un atome d'hydrogène):

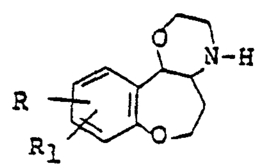

(V)

(2) pour préparer un composé de formule générale (I), dans laquelle $R_2$ représente autre chose qu'un atome d'hydrogène ou un groupe méthyle, l'acylation de ladite benzoxépino-[5,4-*b*]-1,4-oxazine de formule (V) par un chlorure d'acide ou un anhydride d'acide en présence d'une trialkylamine pour préparer une *N*-acyl-(4a,11b)-3,4,4a,5,6,11b-hexahydro-2*H*-[1]benzoxépino-[5,4-*b*]-1,4-oxazine ayant la formule générale (VI):

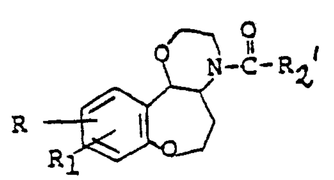

(VI)

dans laquelle $R_2'$ est défini comme $R_2$ ci-dessus, mais contient un groupe méthylène de moins que $R_2$; et la réduction de ladite *N*-acyl-benzoxépino[5,4-*b*]-1,4-oxazine en utilisant un hydrure ou du diborane dans un solvant organique inerte et la récupération de la (4a,11b)-3,4,4a,5,6,11b-hexahydro-2*H*-[1]-benzoxépino[5,4-*b*]-1,4-oxazine voulue, ou

(3) pour préparer un composé de formule générale (I), dans laquelle $R_2$ représente un groupe méthyle, la réaction de ladite benzoxépino[5,4-*b*]-1,4-oxazine de formule (V) avec un chloroformiate d'alkyle en présence d'une trialkylamine, pour préparer un dérivé de type (4a,11b)-3,4,4a,5,6,11b-hexahydro-4-carboalcoxy-2H-[1]-benzoxépino[5,4-*b*]-1,4-oxazine de formule générale VIII

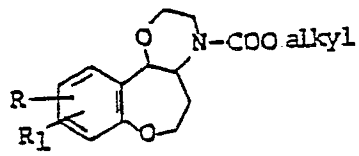

(VIII)

et la réduction dudit dérivé de benzoxépino[5,4-*b*]-1,4-oxazine, en utilisant un hydrure ou du diborane dans un solvant organique inerte, et la récupération du dérivé de 4-méthyle voulu.

2. Procédé selon la revendication 1, pour la production de la *trans*-(4a,11b)-3,4,4a,5,6,11b-hexahydro-10-méthoxy-2*H*-[1]benzoxépino[5,4-*b*]-1,4-oxazine.

3. Procédé selon la revendication 1 pour la production de la *trans*-(4a,11b)-3,4,4a,5,6,11b-hexahydro-9-méthoxy-2*H*-[1]benzoxépino[5,4-*b*]-1,4-oxazine.

4. Procédé selon la revendication 1, pour la production de la *trans*-(4a,11b)-3,4,4a,5,6,11b-hexahydro-8-méthoxy-2*H*-[1]benzoxépino[5,4-*b*]-1,4-oxazine.